# EUROPÄISCHE PATENTSCHRIFT

(11) **EP 2 435 113 B1**
(45) Veröffentlichungstag und Bekanntmachung des Hinweises auf die Patenterteilung: **01.07.2015**
(21) Anmeldenummer: 10721995.8
(22) Anmeldetag: 14.05.2010
(51) Int. Cl.: A61M 5/30, A61M 5/178

(54) **ZUR LAGERUNG VON INJEKTIONSLÖSUNGEN GEEIGNETE ZYLINDER-KOLBEN-EINHEIT FÜR EINEN NADELFREIEN INJEKTOR UND VERFAHREN ZUM BLASENFREIEN AUTOMATISCHEN ODER MANELLEN BEFÜLLEN DER ZYLINDER-KOLBEN-EINHEIT, AUCH BEI ATMOSPHÄRENDRUCK**
CYLINDER-PISTON ASSEMBLY SUITABLE FOR STORAGE OF INJECTION SOLUTIONS FOR NEEDLELESS INJECTOR AND METHOD FOR AUTOMATIC OR MANUAL BUBBLE-FREE FILLING OF CYLINDER-PISTON ASSEMBLY, ALSO AT ATMOSPHERIC PRESSURE
ENSEMBLE CYLINDRE/PISTON APPROPRIÉ À LA CONSERVATION DE SOLUTIONS INJECTABLES POUR INJECTEUR SANS AIGUILLE ET PROCÉDÉ DE REMPLISSAGE MANUEL OU AUTOMATIQUE SANS BULLE DE L'ENSEMBLE CYLINDRE/PISTON, MÊME SOUS PRESSION ATMOSPHÉRIQUE

(30) Priorität: 29.05.2009 DE 102009023334
(43) Veröffentlichungstag der Anmeldung: 04.04.2012
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2010/002975
(87) Internationale Veröffentlichungsnummer: WO 2010/136133

(56) Entgegenhaltungen:
- EP-A1- 1 530 978
- EP-A1- 1 557 190
- EP-A1- 1 875 934
- EP-A2- 0 427 457
- EP-A2- 0 688 570

## Beschreibung

Die Erfindung betrifft eine Wasserdampf und Sauerstoff sperrende, druckstabile Zylinder-Kolben-Einheit für einen nadelfreien Injektor, mit einer in einem Zylinder angeordneten Kammer zur dauerhaften und sterilen Aufnahme einer Injektionslösung, einer Stirnwand mit mindestens einer Düsenbohrung bzw. einem Austrittselement, einem druckstabilen Außenzylinder und einem in der Kammer beweglich angeordneten, Wasserdampf und Sauerstoff sperrenden, druckstabilen Kolben,
- wobei die Kammer mit einer ersten Kammer und einer zweiten konzentrischen Kammer, wobei der Querschnitt der ersten Kammer größer ist als der Querschnitt der zweiten Kammer, und
- wobei jede Düsenbohrung bzw. jedes Austrittselement durch eine Wasserdampf und Sauerstoff sperrende, bei Überdruck öffnende Membrane steril verschlossen ausgebildet ist.
Die Erfindung betrifft darüber hinaus Verfahren zum blasenfreien automatischen oder manuellen Befüllen der Zylinder-Kolben-Einheit, auch bei Atmosphärendruck.

Zylinder-Kolben-Einheiten werden in Injektoren bzw. Einweginjektoren eingesetzt, die beispielsweise aus US 2008/0146997 A1. DE 10 2007 004 211 A1 oder DE 10 2007 008 369 A1 bekannt sind. All diese Zylinder-Kolben-Einheiten sind für eine dauerhafte Lagerung von Injektionslösungen z.B. über ein Jahr ungeeignet, da sie nicht Wasserdampf und Sauerstoff sperren können.

Unter Injektionslösung werden flüssige Arzneimittel verstanden. Im Nachfolgenden sollen die verwendeten Fachbegriffe näher erläutert werden. Dem Fachmann ist der Begriff Arzneimittel bekannt. Hierunter versteht man Stoffe oder Stoffgemische für die Human- oder Tiermedizin. Sie bestehen aus dem oder den pharmazeutischen Wirkstoffen sowie weiteren üblichen Bestandteilen, die diesen Wirkstoff pharmazeutisch verwendbar macht, insbesondere Wasser.

Aus der EP 0 688 570 A2 ist eine Insulinspritze mit auswechselbarer Injektionsnadel und mit der Nadel verbindbar auswechselbarer Insulinampulle, die im Spritzengehäuse aufgenommen ist, bekannt. Die Nadel besteht aus zwei voneinander beabstandeten, im wesentlichen koaxialen Nadelteilen, die durch eine Durchflußsperrvorrichtung in Form einer Membrane voneinander getrennt sind. Der äußere Nadelteil, die Injektionsnadel, ist in einem die Nadel aufnehmenden Körper des Nadelgehäuses befestigt und der innere Nadelteil, die Verbindungsnadel zur Insulinampulle, ist in einem im Nadelgehäuse festgelegten Führungskörper angeordnet. Der Oberbegriff von Anspruch 1 ist basiert auf dem in diesem Dokument offenbarten Gegenstand.

Aus der DE 10 2005 054 600 A1 ist eine Zylinderkolbeneinheit mit einem Zylinder und einem darin geführten Kolben bekannt, wobei der Zylinder und der Kolben eine zumindest zeitweise wirkstoffbefüllbare Kammer umschließen und der Zylinder an seinem vorderen Ende mindestens ein Austrittselement aufweist. Der Querschnitt der Kammer oder der Querschnitt der Zylinderinnenwandung vergrößert sich zumindest bereichsweise von vorne nach hinten. Der Kolben weist zumindest in dem - dem Austrittselement zugewandten - vorderen Bereich eine vordere, elastische Schürze auf, deren vordere Außenkante beim unbelasteten Kolben eine Querschnittsfläche aufspannt, die größer ist als eine Fläche, die von einer Umrisslinie aufgespannt wird, die im Bereich des Übergangs von der Schürze zum schürzentragenden Kolbenabschnitt liegt.

Aus der DE 10 2006 040 888 B3 ist ein Verschlusssystem für Behältnisse zur Aufbewahrung oder Verabreichung von flüssigen, pastösen oder pulverförmigen Stoffen bekannt, das aus einer, mit einem Durchgangsloch ausgestatteten Kappe und einem Verschlusselement besteht. Dabei hält die Kappe das Verschlusselement auf dem Behältnis im Bereich der zu verschließenden Öffnung mittels eines am Behältnis vorhandenen Rastelements kraft- und/oder formschlüssig. Die die Behältnisöffnung umgebende Stirnfläche, auf der das Verschlusselement aufliegt, weist eine Vertiefung auf. Das Verschlusselement ist eine viren-, bakterien- und sporendichte Folie, die auf der Stirnfläche und zumindest bereichsweise über der Vertiefung liegt. Beim Aufsetzen der Kappe ist zwischen dem Verschlusselement und der Vertiefung ein Elastomer- oder Klebstoffring angeordnet, der die Vertiefung ausfüllt.

Aus der DE 10 2006 045 959 B3 ist eine Zylinderkolbeneinheit mit einem Zylinder und einem darin geführten, steril gummiabgedichteten Kolben bekannt, wobei der Zylinder und der Kolben eine zumindest zeitweise wirkstoffbefüllbare Kammer umschließen und der Zylinder an seinem vorderen Ende mindestens ein Austrittselement aufweist. Der in einer hinteren Position ruhende Kolben ist gegenüber dem Zylinder mit einem statischen hinteren Dichtelement steril abgedichtet, wobei beide Dichtelemente in einer Dichtstellung jeweils an der Wandung des Zylinders und jeweils an der Wandung des Kolbens anliegen. Räumlich ist hinter jedem statischen Dichtelement ein, das jeweilige Dichtelement aufnehmender, Parkraum angeordnet. Bei betätigtem Kolben werden die einzelnen statischen Dichtelemente aus ihrer jeweiligen Dichtstellung heraus in eine im Parkraum gelegene Parkstellung überführt, wobei jedes Dichtelement in der Parkstellung entweder nur die Zylinderwandung oder nur die Kolbenwandung kontaktiert. Zwischen den beiden statischen Dichtelementen ist mindestens ein dynamisches kolbenseitiges Dichtelement angeordnet, das zumindest bei betätigtem Kolben an der Zylinderinnenwandung anliegt.

Bei der beschriebenen Zylinder-Kolben-Einheit wird ein Verschlusselement als Kolben im Vakuumsetzverfahren in die vorbefüllte Medikamentenkammer eingesetzt. Im Vakuum wird dabei das Verschlusselement auf den Flüssigkeitsspiegel des in die Kammer eingefüllten Medikamentes aufgesetzt. Dieses Verfahren und die dazu benötigten Vorrichtungen sind teuer und aufwendig.

Ausgehend von diesem Stand der Technik liegen der Erfindung die Aufgaben zugrunde,
- eine Wasserdampf und Sauerstoff sperrende, druckstabile Zylinder-Kolben-Einheit zur dauerhaft sterilen Aufnahme einer Injektionslösung sowie Verfahren zum blasenfreien Befüllen der Zylinder-Kolben-Einheit, auch in einem Tray, anzugeben, bei der der Kolben einfacher und bei Atmosphärendruck in den Zylinder eingebracht werden kann, um die Kosten für die Vorrichtung und das Verfahren zu verringern. Gleichzeitig soll aber auch das Füllen von beispielsweise geringen Stückzahlen per Hand gewährleistet werden, so dass z.B. für die Klinikmuster keine Füllmaschine benötigt wird;
- Unterdosierungen (partielles Eindringen) oder keine Dosierung (Abprallen des Injektionsstrahles) sollen sicher vermieden werden (kein wet-shot);
- der Rückstrom von Injektionslösung aus dem Injektionskanal soll vermieden werden;
- die ab Applikationsvolumina von mehr als 150 Mikroliter mögliche Blasenbildung soll vermieden werden;

Diese Aufgaben werden zunächst durch die Vorrichtung des Patentanspruchs 1 gelöst. Demnach ist die Wasserdampf und Sauerstoff sperrende, druckstabile Zylinder-Kolben-Einheit dadurch gekennzeichnet, dass der Kolben, bestehend aus einem inneren Kolbenkörper und einem Flansch mit einem äußeren, quetschbaren Kolbenring, Wasserdampf und Sauerstoff sperrend, druckstabil und steril schließend, ummantelt ausgebildet ist.

Durch eine derartige Ausgestaltung des Kolbens in einen inneren Kolbenkörper mit Flansch und einen Wasserdampf und Sauerstoff sperrenden, steril dichtenden äußeren Kolbenring, beispielsweise in Form eines U-Profils oder einer Sterildichtung mit einer anderen Form, die den Wasserdampf und Sauerstoff sperrenden Flansch mit überlappt, können unterschiedliche Materialien für den Kolben berücksichtigt werden. Zum Verschließen der Kammer bzw. der zweiten, mit Injektionslösung befüllten Kammer und der nachfolgenden möglichen Lagerung auch bis zu einem Jahr wird der äußere Kolbenring aus einem für Arzneimittel zugelassenen Gummi, z.B. West 4590, gefertigt. Der äußere Kolbenring wird beim Einsetzen in die erste Kammer so gedrückt, dass ein Kanal bzw. eine Strömungskante entsteht, durch den die verdrängte Luft aus der Kammer drucklos entweichen kann. Zwischen dem Kolben und der Injektionslösung verbleibt ein Gaspolster, das vollständig in der zweiten Kammer lokalisiert ist. Dieses Gaspolster wird in einem weiteren Schritt aus der zweiten Kammer entfernt, indem die Kammer auf den Kopf gestellt wird (Luft an Düse) und der innere Kolbenkörper des Kolbens in die zweite Kammer gedrückt wird. Der Außendurchmesser des inneren Kolbenkörpers sowie der Flansch sind auf den Innendurchmesser der zweiten Kammer abgestimmt. Wenn die zweite Kammer vollständig entlüftet ist, befindet sich der innere Kolbenkörper ganz in der zweiten Kammer. Durch eine derartige Ausführung wird die Führung des inneren Kolbenkörpers immer gewährleistet.

Aufgabe 2 wird gelöst durch Gestaltung des Düsenausgangs.
Der Düsenausgang endet in einem erhabenen (hervorstehenden) stumpfen Kegel, so dass die Haut beim Injizieren angepresst wird und die Injektionslösung mit relativ geringem Druck die Haut penetriert. Unterstützend wirkt das darunterliegende Unterhautfettgewebe, das lockereres Gewebe im Vergleich zur Kutis darstellt. Da der Injektionslösungsstrahl nicht frei fliegend auf die Haut prallt, kann er auch nicht von der Oberfläche partiell oder vollständig abprallen. Er wird quantitativ appliziert und ein wet-shot wird vermieden.

Aufgabe 3 wird durch stopfenartiges Verschließen der Haut gelöst.
Da der Düsenausgang durch den Kolben, insbesondere den inneren Kolbenkörper, verschlossen wird, bildet der erhabene stumpfe Kegel einen Stopfen auf der Injektionsstelle. Lässt man ihn wenige Sekunden auf der Haut bis sich der Druck in der Haut durch Verteilen der Injektionslösung abgebaut hat, so kann kein Rückströmen erfolgen.

Aufgabe 4 wird durch ein Mehrdüsenmodell, z.B. vier-Düsenmodell, gelöst, so dass bis 0,6 Milliliter blasenfrei appliziert werden kann. Die Erfahrung lehrt, dass nadelfreie subkutane Injektionen mit Volumina größer 150 Mikroliter eine subkutane. Blasenbildung zur Folge haben. Die hier beispielhaft vorliegende Version weist vier Austrittselemente auf und kann deshalb Injektionsvolumina bis 0,6 Milliliter blasenfrei applizieren.

Beim nachfolgenden Injizieren der Injektionslösung mittels einer Antriebseinheit wird die Zylinder-Kolben-Einheit durch beispielsweise Federhaken der Antriebseinheit aufgenommen. Eine alternative Ausführung besteht darin, die Zylinder-Kolben-Einheit in die Antriebseinheit einzuschrauben, mit einem Flansch- oder Bajonettverschluss zu versehen.

Zum Injizieren wird die verrastete Verschlusskappe mit der übergestülpten Membran abgezogen und der innere Kolbenkörper mit Flansch über die Kolbenstange, betätigt durch die Antriebseinheit (beispielsweise durch eine vorgespannte Feder) bis zum Ende der zweiten Kammer so schnell eingeschoben, dass die Geschwindigkeit des aus der/den Düsen austretenden Flüssigkeitsstrahls ausreichend zur Penetration der Haut ist.

Eine bevorzugte Ausgestaltung sieht dabei vor, den inneren Kolbenkörper mit mindestens einer Dichtlippe auszubilden, wobei die Dichtigkeit zwischen innerem Kolbenkörper und der Wandung der zweiten Kammer mit steigendem Druck zunimmt.

Die oben genannte Aufgabe wird weiterhin durch ein Verfahren, unter Berücksichtigung der soeben beschriebenen Zylinder-Kolben-Einheit gelöst.
Das Verfahren umfasst mindestens folgende Schritte:
- Befüllen einer zweiten Kammer mit einer Injektionslösung, wobei das Volumen der Injektionslösung kleiner ist als das Volumen der zweiten Kammer;
- Einsetzen eines Kolbens, ausgebildet mit beispielsweise einer gequetschten U-Profil Ringdichtung, in eine erste Kammer bis zur Anlage an eine Ringfläche, um die zweite Kammer zu verschließen, wobei in der zweiten Kammer ein Gaspolster verbleibt;
- Umdrehen der Kolben-Zylinder-Einheit um ca. 180° um die horizontale Achse und warten bis das Gaspolster in der zweiten Kammer vollständig nach oben zu mindestens einer Düsenbohrung bzw. einem Austrittselement gestiegen ist;
- Verschieben eines inneren Kolbenkörpers mit Flansch durch eine Kolbenstange aus dem äußeren U-Profil Kolbenring in die zweite Kammer, wobei das Gaspolster durch die Düsenbohrung/-en bzw. Austrittselement/-e aus der zweiten Kammer verdrängt wird und eine Verschlusskappe mit Membrane oder eine separate Membrane als Überdruckventil wirkt, die sich bei Austritt des Gaspolsters von den Düsenbohrung/-en bzw. Austrittselement/-en und ringförmigen Andruckkonturen A, B und C abhebt und nach dem Austritt des Gaspolsters die Düsenbohrung/-en bzw. das/die Austrittselement/-e an den ringförmigen Andruckkonturen A, B und C wieder verschließt (Überdruckventilfunktion).

Durch das Verfahren wird gewährleistet, dass die Zylinder-Kolben-Einheit vollständig gefüllt ist und ohne weitere Maßnahmen separat gelagert oder in einer Antriebseinheit eingesetzt werden kann. Durch die Ausstattung der Verschlusskappe mit einer Membrane ist immer gewährleistet, dass die Injektionslösung dreifach, an den ringförmigen Andruckkonturen A, B und C steril verschlossen bleibt.

Zum händischen Befüllen, z.B. für Klinikversuche wird der Kolben etwa 2 - 3 mm vor die Ringfläche positioniert. Bei nach oben zeigender Düse wird durch den äußeren Kolbenring die Injektionslösung in die Kammer injiziert. Durch Vorschieben des Kolbens bis an die Ringfläche und weiteren Vorschieben des inneren Kolbenkörpers mit Flansch wird die Luft durch das von der Membrane gebildete Überdruckventil verdrängt.

In einer Weiterbildung des Verfahrens wird vorgeschlagen, dass der vollständige Austritt des Gaspolsters ermittelt wird. Hierzu wird/werden beispielsweise die Düsenbohrung/-en bzw. die Austrittselement/-e durch eine Lichtschranke auf das Austreten der Injektionsflüssigkeit hin überwacht. Sobald eine Veränderung erkannt wird, wird das Einschieben des inneren Kolbenkörpers direkt gestoppt.

Da die Luft eine sehr viel geringere Kolbenvorschubkraft benötigt als die Injektionslösung kann das Abschalten des Kolbenvorschubs auch so gesteuert werden.

Bei der heutigen Genauigkeit der Flüssigdosierung und der Präzision von im Spritzguss gefertigten Kammern ist auch der rechnerisch ermittelte Vorschub der Kolbenstange möglich. Bei geringen Stückzahlen erfolgt das Verdrängen der Luftblase unter Sichtkontrolle.

Weitere Merkmale und Einzelheiten der Erfindung ergeben sich aus den Ansprüchen und der nachfolgenden Beschreibung eines in schematischen Zeichnungen dargestellten Ausführungsbeispiels der Erfindung. Es zeigen:
- Fig. 1: in geschnittener Seitenansicht einen Zylinder einer Zylinder-Kolben-Einheit mit einer eingerasteten Verschlusskappe mit Membran;
- Fig. 2: Zylinder der Fig. 1, z.B. im Tray in der Füllmaschine sitzend, nach dem sterilen Abfüllen der Injektionsflüssigkeit nur in die zweite Kammer;
- Fig. 3: Zylinder der Fig. 2 nach dem druckfreien, sterilen Setzen des Kolbens, bestehend aus innerem Kolbenkörper mit Flansch und äußerem Kolbenring in die erste Kammer bündig auf die nicht durch Injektionslösung benetzte Ringfläche und verbleibender Lufteinschluss;
- Fig. 4: in geschnittener Seitensicht die Zylinder-Kolben-Einheit um 180° um die horizontale Achse gedreht, so dass der Lufteinschluss zur Austrittsdüse wandert;
- Fig. 5: in geschnittener Seitenansicht die Zylinder-Kolben-Einheit nach Fig.4, wobei die Injektionslösung die zweite Kammer vollständig ausfüllt; zusätzlich alternative Membranmontage, Federhaken und Auslöser;
- Fig. 6: eine Zylinder-Kolben -Einheit nach Abnahme der Verschlusskappe mit Membrane und getätigter Injektion; Auslöseweg;
- Fig. 7: Befüllung der eindüsigen Zylinder-Kolben-Einheit mit einer Spritze per Hand;
- Fig. 8: Befüllung der mehrdüsigen Zylinder-Kolben-Einheit mit einer Spritze per Hand;
- Fig. 9: in Unteransicht die Stirnwand eines Zylinders mit einer Düse; und
- Fig. 10: in Unteransicht die Stirnwand eines Zylinders mit vier Düsen.

In allen Figuren sind gleiche technische Elemente mit gleichen Bezugszeichen bezeichnet.

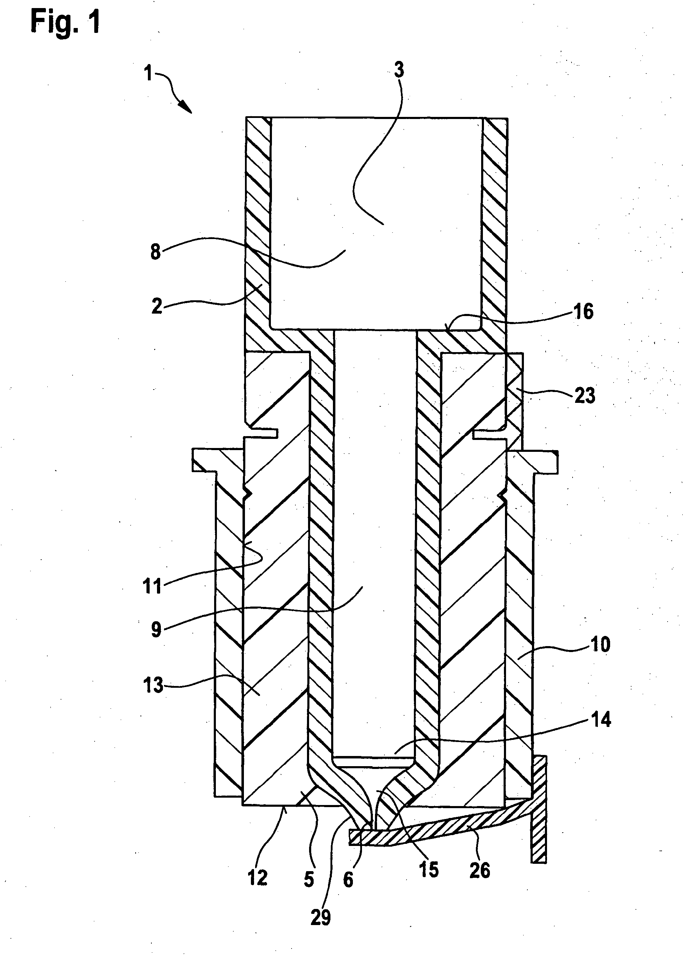

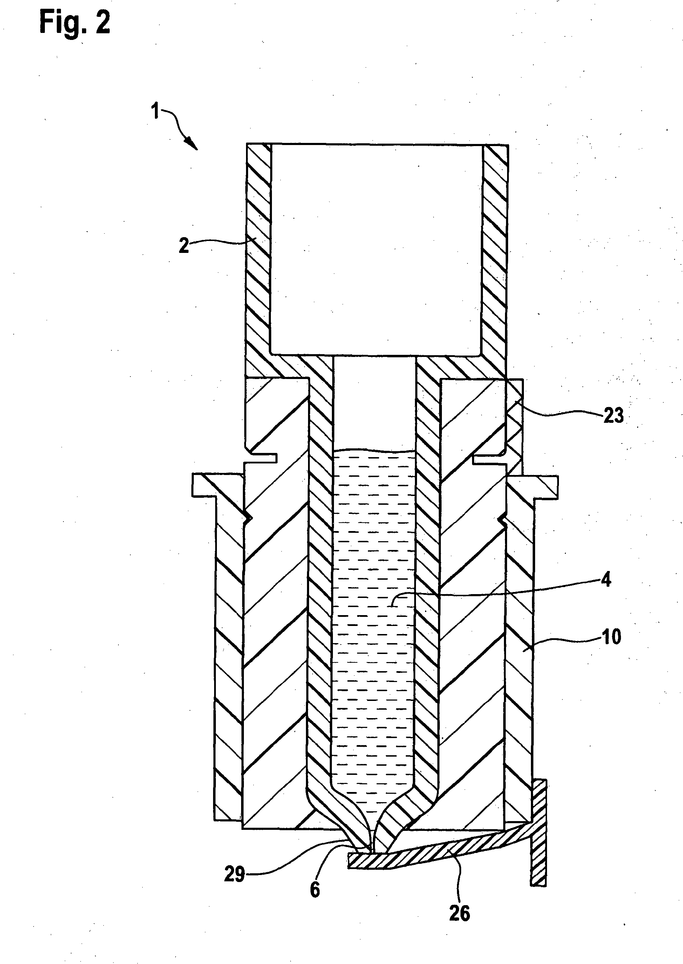

In Figur 1 ist in geschnittener Seitenansicht eine Zylinder-Kolben-Einheit 1 dargestellt. Eine Kammer 3 eines Wasserdampf und Sauerstoff sperrenden Zylinders 2 wird durch eine erste Kammer 8 und eine zweite konzentrische Kammer 9 gebildet, wobei der Querschnitt der ersten Kammer 8 größer ist als der Querschnitt der zweiten Kammer 9. Die zweite Kammer 9 ist an der der ersten Kammer 8 gegenüberliegenden Seite mit einer Stirnwand 5 ausgebildet, die mindestens eine Düsenbohrung bzw. mindestens ein Austrittselement 6 aufweist. Jede Düsenbohrung bzw. jedes Austrittselement 6 endet an der Außenseite 12 des druckstabilen und die zweite Kammer 9 umhüllenden Außenzylinders 13 in einem erhabenen stumpfen Kegel 29 und weist an einer Innenseite 14 der Stirnwand 5 einen Ausströmtrichter 15 auf. Auf der Außenseite 12 des druckstabilen Außenzylinders 13 befindet sich eine über das/die Austrittselemente 6 gespannte und vorzugsweise von der Verschlusskappe 10 getragene Wasserdampf und Sauerstoff sperrende Membrane 26, beispielsweise aus Gummi oder transparentem Silikon. Alternativ kann eine Membrane 27 am Außenzylinder 13 als Sterilverschluss und Überdruckventil befestigt sein.
Zwischen der ersten Kammer 8 und der zweiten Kammer 9 ist ein Übergangsbereich ausgebildet, vorzugsweise als Ringfläche 16. Eine erste Ausgestaltung des Übergangbereiches sieht dabei Radien (nicht dargestellt) beim Übergang von der zylindrischen Wandung der ersten Kammer 8 zur Ringfläche 16 und von der Ringfläche 16 zur zylindrischen Wandung der zweiten Kammer 9 vor. Eine zweite Ausgestaltung dieses Bereiches sieht Schrägen (nicht dargestellt) vor. Weitere Ausgestaltungen, auch Kombinationen von beispielsweise Radien und Schrägen, negativem oder positivem Sturz bzw. konkav oder konvex, können berücksichtigt werden. Die Ausgestaltungen helfen zum Einen beim Einfüllen der Injektionslösung 4, siehe Figur 2, und zum Anderen bei der Herstellung des Zylinders 2, der beispielsweise im Kunststoffspritzgießverfahren oder aus Glas hergestellt wird. Durch die oben beschriebenen Ausgestaltungen wird das Fließverhalten des berücksichtigten Wasserdampf und Sauerstoff sperrenden Materials unterstützt, um einen Zylinder 2 mit den vorgegebenen Eigenschaften wie Rechtwinkligkeit, Rundlauf, Wandstärke usw. zu erhalten.

Die Injektionslösung 4 wird mit bekannten Vorrichtungen in die zweite Kammer 9 derart abgefüllt, dass die zweite Kammer 9 nicht vollständig gefüllt ist. Das Abfüllen erfolgt unter Atmosphärendruck. Das Befüllen erfolgt für jeden Zylinder 2 separat oder in Gruppen, wobei z.B. zwanzig Zylinder in einer Matrix von vier x fünf in einem Tray gleichzeitig befüllt werden.

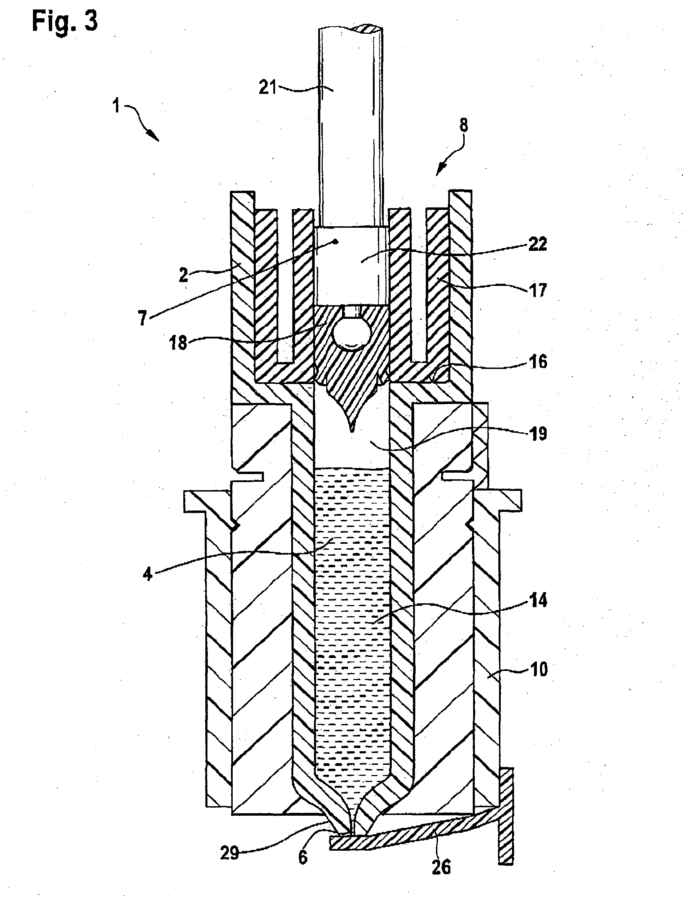

Anschließend wird ein Kolben 7 als mehrteiliges Verschlusselement in die erste Kammer 8 bei Atmosphärendruck eingeschoben, wie in Figur 3 dargestellt. Das Verschlusselement, gebildet aus einem äußeren Kolbenring 17 und einem inneren Kolbenkörper 18 mit einem Wasserdampf und Sauerstoff sperrenden Flansch 22, wird soweit in die erste Kammer 8 eingeschoben, bis ein Kontakt mit der Ringfläche 16 erfolgt. Der äußere Kolbenring 17 wird vom Setzkopf der Füllmaschine seitlich so zusammengequetscht, dass er beim Einsetzen in die erste Kammer 8 keinen Druck auf die in der zweiten Kammer 9 befindliche Injektionslösung 4 ausübt. Verfahren und Vorrichtungen zum drucklosen Einsetzen eines Stopfens bzw. eines Verschlusses sind bekannt. Dabei wird beispielsweise der äußere Kolbenring 17 so verformt, dass das zwischen Verschlusselement und der Injektionslösung 4 befindliche Gas, im vorliegenden Fall Luft, nicht komprimiert wird sondern entweichen kann. Nach Entfernen des Setzkopfes schließt die U-Profil Ringdichtung steril, Wasserdampf und Sauerstoff sperrend ab.

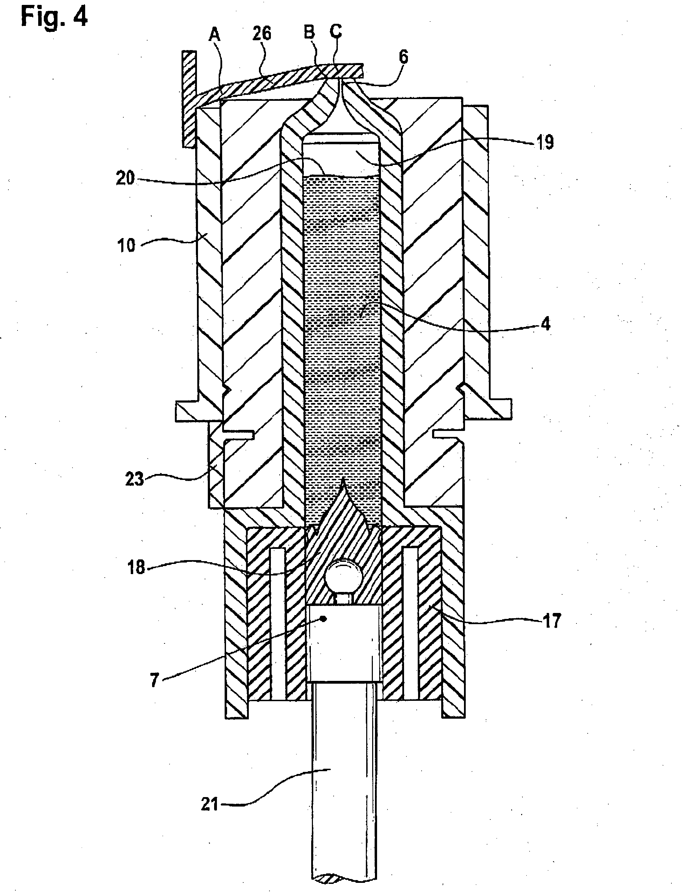

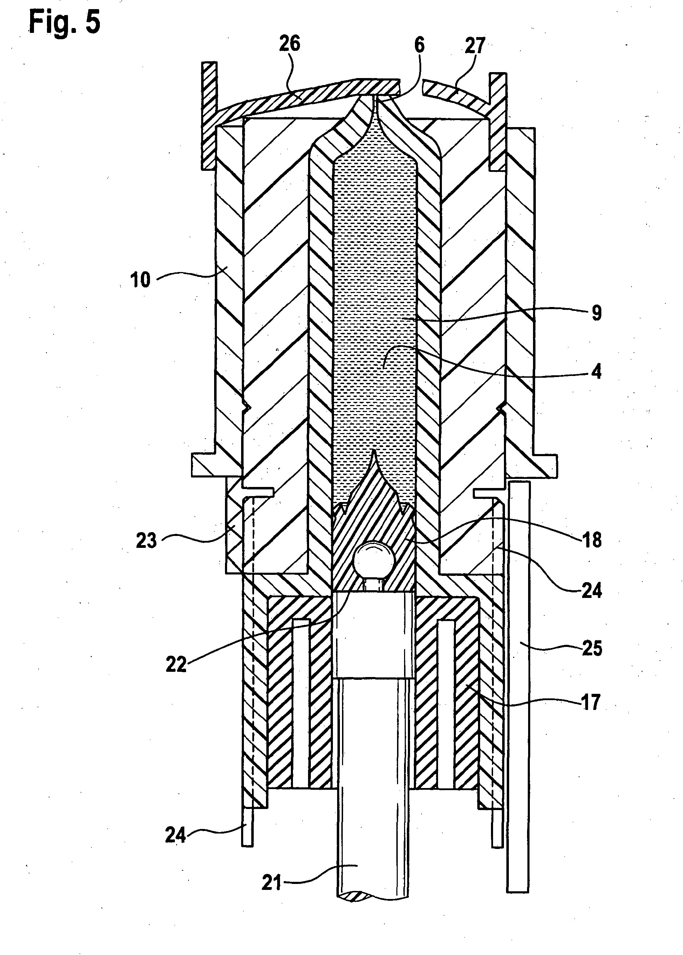

Nach dem Setzen des Kolbens 7, gebildet von dem äußeren Kolbenring 17 und dem inneren Kolbenkörper 18 mit Flansch 22, der auch von einer Kolbenstange 21 getrennt ausgebildet sein kann, befindet sich in der zweiten Kammer 9 eine Gasblase I ein Lufteinschluss 19 und die Injektionslösung 4. Zum Entfernen der Gasblase / des Lufteinschlusses 19 wird die Zylinder-Kolben-Einheit 1 um ca. 180° um die horizontale Achse gedreht. Die Stirnwand 5 mit der aufgesetzten Verschlusskappe 10 mit der Membran 26 zeigt nun nach oben, wie in Figur 4 dargestellt. Die in der zweiten Kammer 9 befindliche Gasblase / Lufteinschluss19 steigt nach oben und befindet sich zwischen der Oberkante 20 der Injektionslösung 4 und der Düsenbohrung / -en bzw. der Austrittsöffnung / -en 6. Wenn sich die Gasblase 19 vollständig oberhalb der Oberkante 20 der Injektionslösung 4 befindet - die hierfür notwendige Zeit ist u.a. abhängig von der Viskosität der Injektionslösung 4, der Umgebungstemperatur usw. und wird bei den weiteren Verfahrensschritten berücksichtigt - wird der innere Kolbenkörper 18 des Verschlusselementes mittels der Kolbenstange 21 und dem Flansch 22 nach oben bewegt. Dabei verbleibt der äußere Kolbenring 17 ortsfest an der Ringfläche 16 anliegend. Der innere Kolbenkörper 18 wird mit Flansch 22 soweit in die zweite Kammer 9 hinein geschoben, bis die Gasblase / der Lufteinschluss 19 vollständig durch die Düsenbohrung / -en bzw. die Austrittsöffnung / -en 6 aus der zweiten Kammer 9 entwichen ist, wie in Figur 5 dargestellt. Dazu hebt sich eine Membran 26 der Verschlusskappe 10 bei einem Überdruck in der zweiten Kammer 9 von den ringförmigen Andruckkonturen A, B und C. Dabei passiert die sterile Luft die sterile Membran 26 und entweicht durch den Spalt zwischen Zylinder und glasklarer Verschlusskappe 10. Das vollständige Herausdrücken der Gasblase / des Lufteinschlusses 19 aus der zweiten Kammer 9 wird mit an sich bekannten Messverfahren und Messvorrichtungen (Lichtschranke) dadurch ermittelt, dass die Injektionslösung 4 in der/den Düsenbohrung / -en bzw. der Austrittsöffnung / -en 6 erkannt wird. Eine andere Variante besteht darin, den inneren Kolbenkörper 18 bis zu einer vorbestimmten Position in die zweite Kammer 9 zu schieben. Bei einer weiteren Variante wird der Vorschub elektronisch gesteuert abgeschaltet, wenn sich die Kolbenvorschubkraft schlagartig nach dem Entweichen der Luft erhöht.

Wie in Figur 5 dargestellt, ist die Kolbenstange 21 mit einem Flansch 22 ausgebildet, der Flansch 22 kann aber auch von der Kolbenstange 21 getrennt ausgebildet sein. Bei einer voll befüllten zweiten Kammer 9 ist die Kante des äußeren Kolbenringes 17 bündig mit dem Flansch 22 der Kolbenstange 21. Der Durchmesser der vorgespannten Dichtlippe des inneren Kolbenkörpers 18 ist so gewählt, dass sie mit steigendem Druck stärker dichtend beim Ausstoßen der Injektionslösung 4 aus der zweiten Kammer 9 in diese eintauchen kann. Der druckstabile Außenzylinder 13 ist an der Mantelfläche 11 mit wahlweise Halteelementen 23 wie Schlitz, Gewinde, Flansch oder Bajonett ausgebildet. Komplementär zu der Form der Haltelemente 23 ist die Antriebseinheit des Einweginjektors ausgebildet. Zum Ausstoßen der Injektionslösung wird der innere Kolbenkörper 18 mit Flansch 22 durch die Kolbenstange 21 in Richtung auf die Stirnwand 5 bewegt. Dieses geschieht beispielsweise durch eine vorgespannte Feder, wie in den o.g. Druckschriften zu Einweginjektoren genauer beschrieben. Zylinder 2 und Flansch 22 müssen bei einjähriger Lagerzeit der mit Injektionslösung 4 befüllten Zylinder-Kolben-Einheit Wasserdampf und Sauerstoff sperrend sein.

Als Werkstoffe für den Zylinder 2 und den Flansch 22 eignen sich neben Glas transparente, amorphe Thermoplaste, z.B. ein Copolymer auf der Basis von Cycloolefinen und Ethylenen oder α-Olefinen (COC bzw. COP).

Der Außenzylinder 13 muss kurzzeitig (wenige Millisekunden) bis zu 350 bar Druck vertragen und deshalb aus einem druckstabilen Material, beispielsweise Polycarbonat (PC) oder Polyurethan (PU) bestehen.

Für den inneren Kolbenkörper 18 wird als Werkstoff ein Tetrafluorehtylen/Hexafluorpropylen-Copolymer (FEP) verwendet. Dieser hat in Verbindung mit den zuvor genannten Werkstoffen des Zylinders 2 bzw. Innenteils selbstschmierende Eigenschaften, so dass zwischen dem inneren Kolbenkörper 18 und dem Zylinder 2 keine separaten Schmiermittel benötigt werden. Als alternative Werkstoffe stehen u.a. Perfluoralkoxy-Copolymer (PFA), Tetrafluorehtylen (E TFE) oder Polyvinylidenfluorid (PVDF) zur Auswahl.

Für dem äußeren Kolbenring 17 wird als Werkstoff ein Gummi wie Helveot FM 257, Helvoet FM 460, Stelmi 6422, Stelmi 6720 oder West 4590 verwendet. Aus den gleichen Wasserdampf und Sauerstoff sperrenden, steril schließenden Materialien kann die Membrane 26 gefertigt sein.

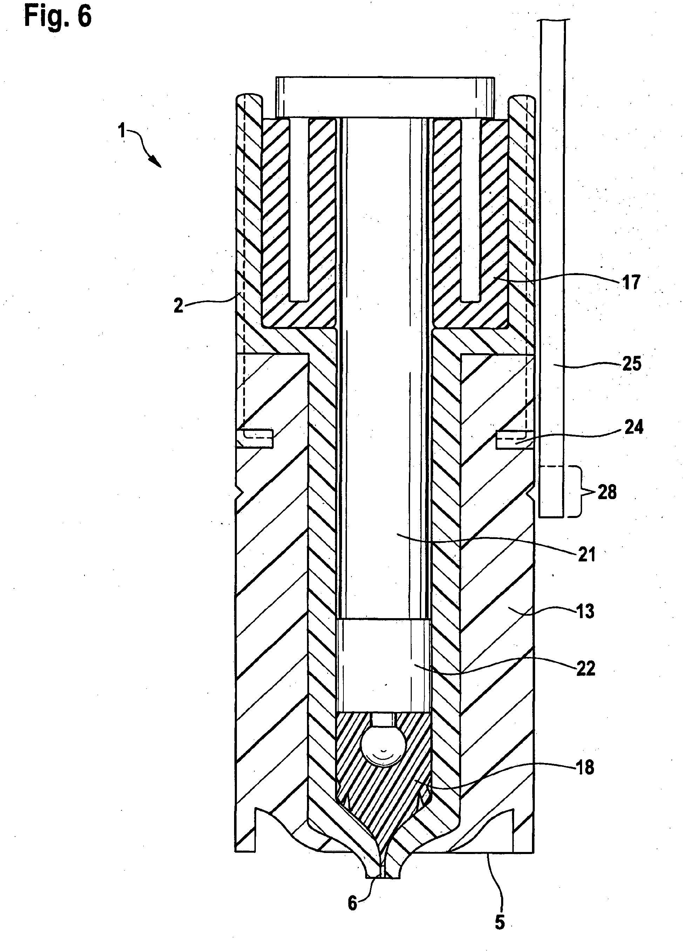

Figur 6 zeigt eine Zylinder-Kolben-Einheit 1 nach Abnahme der Verschlusskappe mit Membrane 26 und getätigter Injektion. Links ist eine alternative Formgebung der Außenseite der Stirnwand 12 dargestellt. Durch die Erhabenheit des die Düse 6 umgebenden Kegels 29 wird die Haut angepresst und damit leichter vom Injektionsstrahl durchschossen. Die Injektionslösung 4 breitet sich dann im, im Vergleich zur Kutis, lockereren Unterhautfettgewebe aus. Auf diese Weise wird eine quantitative Applikation sichergestellt. Da die Düse 6 mit dem untenstehenden inneren Kolbenkörper 18 verschlossen ist wirkt der stumpfe Kegel 29 wie ein Deckel auf den Hautkanal, so dass ein Rückströmen der Injektionslösung verhindert wird. Nach wenigen Sekunden hat sich die unter Druck stehende Injektionslösung im lockereren Unterhautfettgewebe verteilt und der Injektionskanal in der Haut zusammengezogen. Dann kann der Injektor von der Injektionsstelle entfernt werden.

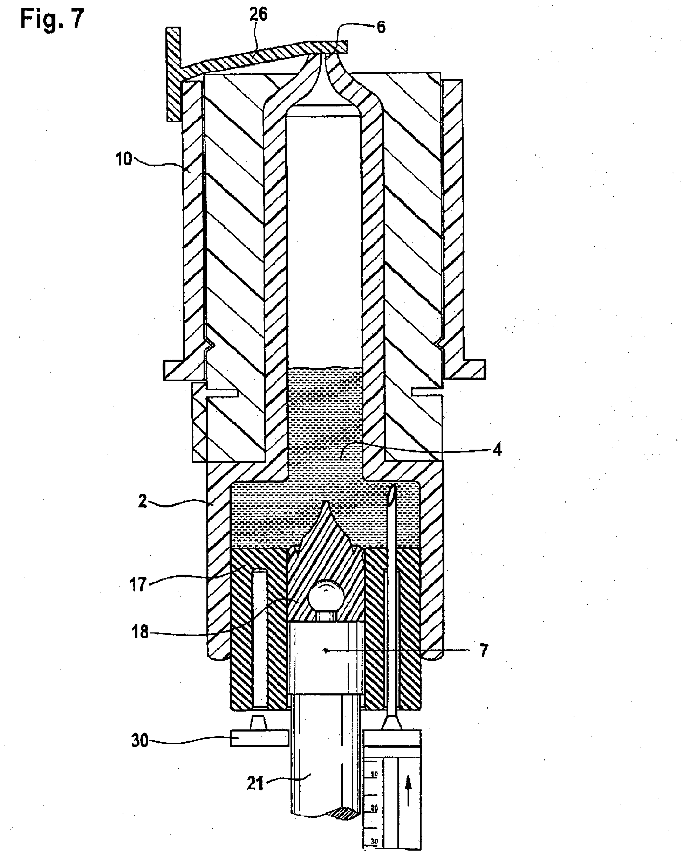

Figur 7 zeigt die sterile Befüllung per Hand, z.B. für eine klinische Prüfung, ohne eine aufwendige Füllmaschine. Dazu wird werkseitig die Einheit Kolben 7, bestehend aus innerem Kolbenkörper 18 mit Flansch 22 und durch einen Profilring 30 verschlossener äußerer U-Profilkolbenring 17 in die erste Kammer 8 nur bis 2 - 3 mm vor die Ringfläche 16 platziert. Diese Zylinder-Kolben-Einheit 1 wird dann Gamma - steritisiert. In der Klinik kann dann der steril schließende Profilring 30 abgezogen werden. In den Hohlraum des U-Profils 17 kann nun die Kanüle einer mit Injektionslösung gefüllten Spritze eingeführt werden, das U-Profilgummi 17 durchstochen werden und der Rest der ersten Kammer 8 und ein Teil der zweiten Kammer 9, mit der Düse 6 in oberer Position, befüllt werden. Nach Entfernen der Injektionsnadel wird die Einheit Kolben 7, bestehend aus innerem Kolbenkörper 18 mit Flansch 22 und äußerer U-Profil Kolbenring 17 bis zur Ringfläche 16 vorgeschoben, damit dann die vor der Düse 6 verbliebene Luft 19 wie bei Figur 5 verdrängt wird. Nach der Verbindung mit der Antriebseinheit ist das System fertig zur Lagerung oder Applikation.

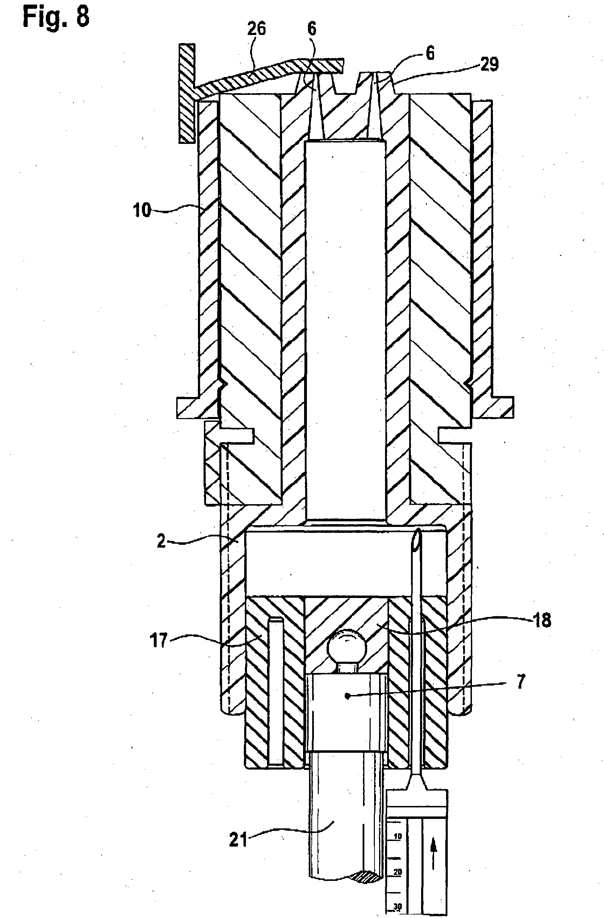

Auf die gleiche Weise wie in Figur 7 dargestellt, lässt sich auch eine mehrdüsige Zylinder-Kolben-Einheit per Hand befüllen, wie in Figur 8 dargestellt. Die maschinelle Befüllung verläuft analog zur eindüsigen Version.

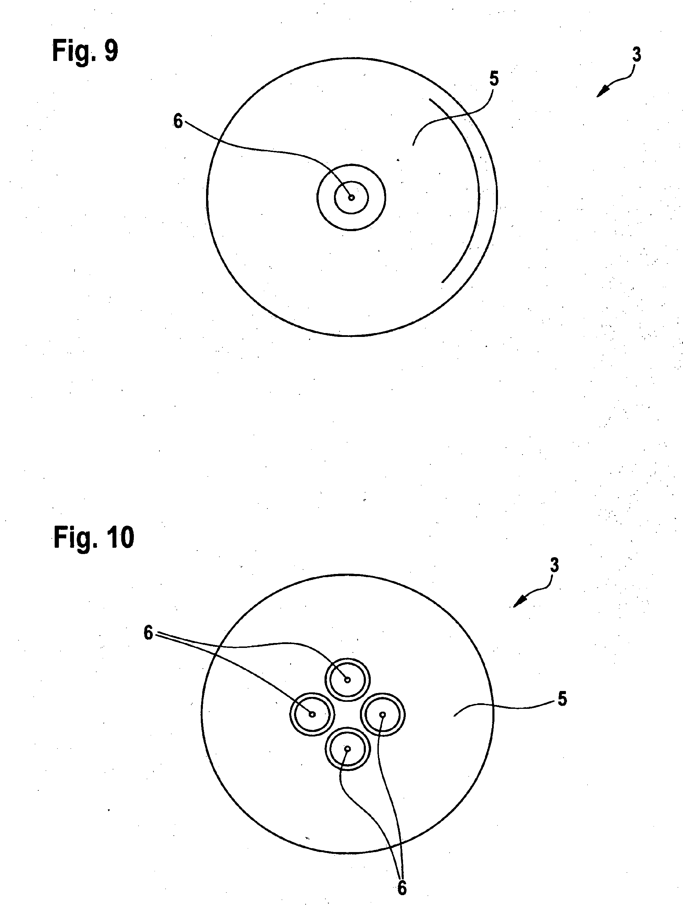

Figur 9 zeigt in Unteransicht eine Version des Zylinders 3 mit Stirnwand 5 und einer Düse 6.

Figur 10 zeigt in Unteransicht eine Version des Zylinders 3 mit Stirnwand 5 und vier Düsen 6.

### Bezugszeichenliste

- 1.: Zylinder-Kolben-Einheit
- 2.: Zylinder, Wasserdampf und Sauerstoff sperrend
- 3.: Kammer
- 4.: Injektionslösung
- 5.: Stirnwand
- 6.: Düsenbohrung bzw. Austrittselement
- 7.: Kolben, bestehend aus äußerem Kolbenring 17, innerem Kolbenkörper 18 und Flansch 22
- 8.: erste Kammer
- 9.: zweite Kammer
- 10.: Verschluss
- 11.: Mantelfläche
- 12.: Außenseite Stirnwand
- 13.: Außenzylinder, druckstabil
- 14.: Innenseite der Kammer bzw. des Ausströmtrichters
- 15.: Ausströmtrichter
- 16.: Ringfläche
- 17.: äußerer Kolbenring, U-Profilring
- 18.: innerer Kolbenkörper
- 19.: Gasblase / Lufteinschluss
- 20.: Oberkante
- 21.: Kolbenstange
- 22.: Flansch der Kolbenstange, auch getrennte Ausführung
- 23.: Halteelement
- 24.: Federhaken
- 25.: Auslöser, Auslöserohr
- 26.: Membrane an Verschluss 10
- 27.: Membrane an Zylinder, alternativ
- 28.: Auslöseweg
- 29.: Kegel, stumpf
- 30.: Profilring
- A, B, C: ringförmige Andruckkonturen

## Patentansprüche

1. Wasserdampf und Sauerstoff sperrende, druckstabile Zylinder-Kolben-Einheit (1) für einen nadelfreien Injektor, mit einer in einem Zylinder (2) angeordneten Kammer (3) zur dauerhaften und sterilen Aufnahme einer Injektionslösung (4), einer Stirnwand (5) mit mindestens einer Düsenbohrung bzw. einem Austrittselement (6), einem druckstabilen Außenzylinder (13) und einem in der Kammer (3) beweglich angeordneten, Wasserdampf und Sauerstoff sperrenden, druckstabilen Kolben (7),
- wobei die Kammer (3) mit einer ersten Kammer (8) und einer zweiten konzentrischen Kammer (9), wobei der Querschnitt der ersten Kammer (8) größer ist als der Querschnitt der zweiten Kammer (9), und
- wobei jede Düsenbohrung bzw. jedes Austrittselement (6) durch eine Wasserdampf und Sauerstoff sperrende, bei Überdruck öffnende Membrane (26, 27) steril verschlossen ausgebildet ist,
**dadurch gekennzeichnet,**
**dass** der Kolben (7), bestehend aus einem inneren Kolbenkörper (18) und einem Flansch (22) mit einem äußeren, quetschbaren Kolbenring (17), Wasserdampf und Sauerstoff sperrend, druckstabil und steril schließend, ummantelt ausgebildet ist.

2. Zylinder-Kolben-Einheit nach Anspruch 1,
**dadurch gekennzeichnet,**
**dass** die Membrane (26) mit einem Verschluss (10) verbunden oder die Membrane (27) separat entfernbar als Sterilverschluss und Überdruckventil ausgebildet ist.

3. Zylinder-Kolben-Einheit nach Anspruch 2,
**dadurch gekennzeichnet,**
**dass** die Verschlusskappe (10) direkt oder indirekt mit einer Mantelfläche (11) oder einer Außenseite der Stirnwand (12) des Zylinders (13) oder einem Auslöserohr (25) lösbar verbunden ausgebildet ist.

4. Zylinder-Kolben-Einheit nach einem der Ansprüche 1 bis 3,
**dadurch gekennzeichnet,**
**dass** zwischen der ersten Kammer (8) und der zweiten Kammer (9) ein Übergangsbereich als Ringfläche (16) ausgebildet ist.

5. Zylinder-Kolben-Einheit nach einem der Ansprüche 1 bis 4,
**dadurch gekennzeichnet,**
**dass** der innere Kolbenkörper (18) und der Flansch (22) der Kolbenstange (21) mit dem äußeren Kolbenring (17) und dieser mit dem Zylinder (2) steril dichtend und Wasserdampf sowie Sauerstoff sperrend ausgebildet sind.

6. Zylinder-Kolben-Einheit nach einem der Ansprüche 1 bis 5,
**dadurch gekennzeichnet,**
**dass** der innere Kolbenkörper (18) an der der zweiten Kammer (9) zugewandten Kante mit mindestens einer mit steigendem Druck dichter schließenden Dichtlippe ausgebildet ist.

7. Verfahren zum Befüllen einer Zylinder-Kolben-Einheit (1) für einen nadelfreien Einweginjektor, umfassend mindestens folgende Schritte:
- Befüllen einer zweiten Kammer (9) mit einer Injektionslösung (4), wobei das Volumen der Injektionslösung (4) kleiner ist als das Volumen der zweiten Kammer (9);
- Einsetzen eines Kolbens (7) mit gequetschtem äußeren Kolbenring (17), so dass die Luft vorbeiströmt, in eine erste Kammer (8) bis zur Anlage an eine Ringfläche (16), um die zweite Kammer (9) zu verschließen, wobei in der zweiten Kammer (9) ein Gaspolster verbleibt;
- Umdrehen der Kolben-Zylinder-Einheit um ca. 180° um die horizontale Achse und warten bis das Gaspolster in der zweiten Kammer (9) vollständig nach oben zu mindestens einer Düsenbohrung bzw. einem Austrittselement (6) gestiegen ist;
- Verschieben eines inneren Kolbenkörpers (18) mit Flansch (22) durch eine Kolbenstange (21) aus dem äußeren Kolbenring (17) in die zweite Kammer (9), wobei das Gaspolster durch die Düsenbohrung/-en bzw. Austrittselement/-e (6) aus der zweiten Kammer (9) verdrängt wird und die Membrane (26) einer Verschlusskappe (10) oder eine separate Membrane (27) sich bei Austritt des Gaspolsters von den Düsenbohrung/-en bzw. Austrittselement/-en (6) und ringförmigen Andruckkonturen A B und C abhebt und nach dem Austritt des Gaspolsters die Düsenbohrung/-en bzw. das/die Austrittselement/-e (6) an den ringförmigen Andruckkonturen A, B und C wieder steril verschließt.

8. Verfahren nach Anspruch 7,
**dadurch gekennzeichnet,**
**dass** der vollständige Austritt des Gaspolsters ermittelt wird, indem die Düsenbohrung/-en bzw. die Austrittselement/-e durch eine Lichtschranke auf das Austreten der Injektionsflüssigkeit hin überwacht werden.

9. Händisches Befüllen einer Zylinder-Kolben-Einheit nach einem der Ansprüche 1 bis 6, indem der Kolben (7) 2 - 3 mm vor die Ringfläche (16) positioniert wird; bei nach oben zeigender Düse (6) wird durch den äußeren Kolbenring (17) die Injektionslösung (4) in die Kammer (3) injiziert; und
durch Vorschieben des Kolbens (7) bis an die Ringfläche (16) und weiterem Vorschieben des inneren Kolbenkörpers (18) die Luft durch das Überdruckventil, gebildet von der Membrane (26) oder der Membrane (27), verdrängt wird.

## Claims

1. Pressure-stable cylinder/piston unit (1) which blocks water vapor and oxygen and is designed for a needle-free injector, with a chamber (3) arranged in a cylinder (2) and designed for long-term and sterile storage of an injection solution (4), an end wall (5) with at least one nozzle bore or one outlet element (6), a pressure-stable outer cylinder (13), and a pressure-stable piston (7) arranged movably in the chamber (3) and blocking water vapor and oxygen,
- wherein the chamber (3) is designed with a first chamber (8) and a second, concentric chamber (9), wherein the cross section of the first chamber (8) is greater than the cross section of the second chamber (9), and
- wherein each nozzle bore or each outlet element (6) is closed in a sterile manner by a membrane (26, 27) that blocks water vapor and oxygen and that opens at an overpressure,
**characterized in that** the piston (7), consisting of an inner piston body (18) and a flange (22) with an outer, squeezable piston ring (17), is designed such that it blocks water vapor and oxygen, is pressure-stable and closes in a sterile manner.

2. Cylinder/piston unit according to Claim 1, **characterized in that** the membrane (26) is connected to a closure piece (10), or the membrane (27) is designed separately removable as a sterile closure piece and overpressure valve.

3. Cylinder/piston unit according to Claim 2, **characterized in that** the closure cap (10) is designed to be releasably connected directly or indirectly to a jacket surface (11) or to an outside of the end wall (12) of the cylinder (13) or to a trigger tube (25).

4. Cylinder/piston unit according to one of Claims 1 to 3, **characterized in that** a transition area between the first chamber (8) and the second chamber (9) is designed as an annular surface (16).

5. Cylinder/piston unit according to one of Claims 1 to 4, **characterized in that** the inner piston body (18) and the flange (22) of the piston rod (21) are designed, along with the outer piston ring (17), and the latter along with the cylinder (2), to provide a sterile seal and block water vapor and oxygen.

6. Cylinder/piston unit according to one of Claims 1 to 5, **characterized in that** the inner piston body (18), at the edge directed toward the second chamber (9), is designed with at least one sealing lip that closes more tightly as the pressure increases.

7. Method for filling a cylinder/piston unit (1) for a needle-free injector, comprising at least the following steps:
- filling a second chamber (9) with an injection solution (4), wherein the volume of the injection solution (4) is smaller than the volume of the second chamber (9) ;
- inserting a piston (7) with squeezed outer piston ring (17), such that the air flows past, into a first chamber (8) until it bears on an annular surface (16) in order to close the second chamber (9), wherein a gas cushion remains in the second chamber (9);
- rotating the piston/cylinder unit through ca. 180° about the horizontal axis and waiting until the gas cushion in the second chamber (9) has risen fully upward to at least one nozzle bore or one outlet element (6);
- moving an inner piston body (18) with flange (22), by means of a piston rod (21), out of an outer piston ring (17) into the second chamber (9), wherein the gas cushion is forced through the nozzle bore(s) or outlet element(s) (6) from the second chamber (9), and, as the gas cushion emerges, the membrane (26) of a closure cap (10), or a separate membrane (27), lifts away from the nozzle bore(s) or outlet element(s) (6) and annular pressure contours A, B and C, and, after the emergence of the gas cushion, again closes the nozzle bore(s) or outlet element(s) (6) on the annular pressure contours A, B and C in a sterile manner.

8. Method according to Claim 7, **characterized in that** the complete emergence of the gas cushion is detected by the nozzle bore(s) or outlet element(s) being monitored by a light barrier for the emergence of the injection liquid.

9. Manual filling of a cylinder/piston unit according to one of Claims 1 to 6, with the piston (7) being positioned 2 - 3 mm in front of the annular surface (16) ;
with the nozzle (6) pointing upward, the injection solution (4) is injected through the outer piston ring (17) into the chamber (3); and
by advancing the piston (7) as far as the annular surface (16), and further advancing the inner piston body (18), the air is forced through the overpressure valve formed by the membrane (26) or the membrane (27).

## Revendications

1. Ensemble cylindre-piston (1) stable sous pression, arrêtant la vapeur d'eau et l'oxygène, pour un injecteur sans aiguille, avec une chambre (3) disposée dans un cylindre (2) pour la conservation durable et stérile d'une solution injectable (4), une paroi frontale (5) avec au moins un alésage de buse ou un élément de sortie (6), un cylindre extérieur stable à la pression (13) et un piston stable à la pression (7), disposé de façon mobile dans la chambre (3) et arrêtant la vapeur d'eau et l'oxygène,
- dans lequel la chambre (3) est formée avec une première chambre (8) et une deuxième chambre concentrique (9), dans lequel la section transversale de la première chambre (8) est plus grande que la section transversale de la deuxième chambre (9), et
- dans lequel chaque alésage de buse ou chaque élément de sortie (6) est fermé de façon stérile par une membrane (26, 27) arrêtant la vapeur d'eau et l'oxygène et s'ouvrant en cas de surpression,
**caractérisé en ce que** le piston (7), se composant d'un corps de piston intérieur (18) et d'une bride (22), est entouré avec une bague de piston extérieure écrasable (17), de façon à arrêter la vapeur d'eau et l'oxygène et à se fermer de façon stable à la pression et stérile.

2. Ensemble cylindre-piston selon la revendication 1, **caractérisé en ce que** la membrane (26) est reliée à une fermeture (10) ou la membrane (27) est réalisée de façon apte à être enlevée séparément sous forme de fermeture stérile et de soupape de surpression.

3. Ensemble cylindre-piston selon la revendication 2, **caractérisé en ce que** la coiffe de fermeture (10) est assemblée de façon amovible directement ou indirectement à une surface latérale (11) ou à un côté extérieur de la paroi frontale (12) du cylindre (13) ou à un tube de déclenchement (25)

4. Ensemble cylindre-piston selon l'une quelconque des revendications 1 à 3, **caractérisé en ce qu'**une zone de transition entre la première chambre (8) et la deuxième chambre (9) est réalisée sous forme de face annulaire (16).

5. Ensemble cylindre-piston selon l'une quelconque des revendications 1 à 4, **caractérisé en ce que** le corps de piston intérieur (18) et la bride (22) de la tige de piston (21) sont réalisés sous forme d'étanchéité stérile avec la bague de piston extérieure (17) et celle-ci avec le cylindre (2) et de façon à arrêter la vapeur d'eau et l'oxygène.

6. Ensemble cylindre-piston selon l'une quelconque des revendications 1 à 5, **caractérisé en ce que** le corps de piston intérieur (18) est réalisé sur l'arête tournée vers la deuxième chambre (9) avec une lèvre d'étanchéité se fermant de façon de plus en plus étanche lorsque la pression augmente.

7. Procédé de remplissage d'un ensemble cylindre-piston (1) pour un injecteur jetable sans aiguille, comprenant au moins les étapes suivantes:
- remplissage d'une deuxième chambre (9) avec une solution injectable (4), dans lequel le volume de la solution injectable (4) est plus petit que le volume de la deuxième chambre (9);
- introduction d'un piston (7) avec une bague de piston extérieure écrasée (17), de sorte que l'air s'écoule le long de celle-ci, dans une première chambre (8) jusqu'à l'appui sur une face annulaire (16), afin de fermer la deuxième chambre (9), dans lequel il reste un coussin de gaz dans la deuxième chambre (9);
- rotation de l'ensemble cylindre-piston de 180° autour de l'axe horizontal et attente jusqu'à ce que le coussin de gaz dans la deuxième chambre (9) soit monté entièrement vers le haut jusqu'au moins un alésage de buse ou un élément de sortie (6) ;
- déplacement du corps de piston intérieur (18) avec la bride (22) au moyen d'une tige de piston (21) hors de la bague de piston extérieure (17) dans la deuxième chambre (9), dans lequel le coussin de gaz est chassé hors de la deuxième chambre (9) à travers le/les alésage(s) de buse ou le/les élément(s) de sortie (6) et la membrane (26) d'une coiffe de fermeture (10) ou une membrane séparée (27) se soulève du/des alésage (s) de buse ou du/des éléments de sortie (6) et des contours de pression annulaires A, B et C lors de la sortie du coussin de gaz et ferme de nouveau de façon stérile le/les alésage(s) de buse ou le/les éléments de sortie (6) sur les contours de pression annulaires A, B et C après la sortie du coussin de gaz.

8. Procédé selon la revendication 7, **caractérisé en ce que** l'on détecte la sortie complète du coussin de gaz, du fait que l'on surveille le/les alésage (s) de buse ou le/les élément(s) de sortie au moyen d'une barrière lumineuse pour la sortie du liquide injectable.

9. Remplissage manuel d'un ensemble cylindre-piston selon l'une quelconque des revendications 1 à 6, dans lequel on positionne le piston (7) 2-3 mm devant la face annulaire (16);
on injecte la solution injectable (4) dans la chambre (3) à travers la bague de piston extérieure (17) avec la buse (6) orientée vers le haut; et
on chasse l'air à travers la soupape de surpression, formée par la membrane (26) ou la membrane (27), en faisant avancer le piston (7) jusqu'à la face annulaire (16) et en faisant avancer davantage le corps de piston intérieur (18).
